# EUROPEAN PATENT APPLICATION

(11) **EP 4 365 242 A1**
(43) Date of publication of application: **08.05.2024**
(21) Application number: 22833597.2
(22) Date of filing: 28.06.2022
(51) Int. Cl.: C09C 3/06, C09C 3/00, C09C 1/36, C09D 5/36, C09D 11/037

(54) **MULTI-COLOR PEARLESCENT PIGMENT WITH IMPROVED SPARKLING EFFECT AND PREPARATION METHOD THEREFOR**

(30) Priority: 28.06.2021 KR 20210083971
(71) Applicant: Cqv Co., Ltd., Jincheon-gun, Chungcheongbuk-do 27845 (KR)
(72) Inventor: JEONG, Jae Il, Jincheon-gun Chungcheongbuk-do 24845 (KR); KANG, Kwang Choong, Jincheon-gun Chungcheongbuk-do 24845 (KR); CHOI, Byung Ki, Jincheon-gun Chungcheongbuk-do 24845 (KR); LIM, Kwang Soo, Jincheon-gun Chungcheongbuk-do 24845 (KR); CHANG, Kil Wan, Jincheon-gun Chungcheongbuk-do 24845 (KR)
(74) Representative: AWA Sweden AB
(86) International application number: PCT/KR2022/009248
(87) International publication number: WO 2023/277541

(57) **Abstract**

Disclosed are a pearlescent pigment and a preparation method therefor, wherein the pigment has a multilayered structure including a low-refractive index material layer between high-refractive index material layers on a glass flake substrate and thus has high color intensity, various colors depending on the viewing angle, and an improved sparkling effect. The pearlescent pigment according to the present invention comprises: a glass flake substrate having a D₁₀ value of 40-80µm, a D₅₀ value of 160-250µm, and a D₉₀ value of 350-600µm and a thickness of 500 nm or more; and a metal oxide layer coated on the substrate, the metal oxide layer having a structure of a first metal oxide layer/an intermediate oxide layer containing MgO·SiO₂/a second metal oxide layer.

## Description

### FIELD

The present disclosure relates to a pearlescent pigment, and more specifically, to a pearlescent pigment having high color intensity and various colors depending on a viewing angle using differences in a refractive index of multiple metal oxide layers coated on a platelet-shaped substrate and having an improved sparkling effect because of physical properties of the substrate, and a method for preparing the same.

### DESCRIPTION OF RELATED ART

A pearlescent pigment is used in various fields of industry, especially in fields of automobiles, decorative coatings, plastics, paints, printing inks and in cosmetic formulations.

A 'high-contrast', glossy pigment based on a transparent platelet-shaped substrate without metallic gloss is coated with a high-refractive index metal oxide layer (e.g., TiO₂) and an optional absorbing layer on a mica platelet. When observed in a flat state, such pigment exhibits a specific interference color that depends on a thickness of the TiO₂ layer. The interference color fades and eventually turns gray or black as a viewing angle becomes smaller. At this time, although the interference color does not change, it is observed that a color saturation is lowered. In other words, a range of a color change based on the change in the observation viewing angle is narrow.

Recently, a pearlescent pigment based on glass platelets or mica particles coated with layers of SiO₂ and TiO₂ alternating with an opaque metal layer has been developed.

However, existing known multi-layer pigments may be in some cases made of layer materials that transmit almost no light or transmit a small amount of light, and therefore, may be combined with absorbing pigments only in a very limited range when being applied. In addition, interference colors of such pigments are highly dependent on the viewing angle, which is undesirable in most applications. In addition, in some cases, it is very difficult to prepare or regenerate such pigments.

In addition, pigments using the platelet-shaped substrate, which are widely used in the current market, have a problem of lack of optical properties, especially, sparkling effects, and have a problem of not being able to render various colors because of the narrow range of the color change based on the change in the observation viewing angle as described above.

### DISCLOSURE

### TECHNICAL PURPOSES

A purpose of the present disclosure is to provide a novel pearlescent pigment with excellent optical properties such as sparkling and a wide range of color change based on a change in an observation viewing angle, and a method for preparing the same.

### TECHNICAL SOLUTIONS

A pearlescent pigment according to one embodiment of the present disclosure for achieving the above purpose includes: a glass flake substrate;
a first metal oxide layer coated on the substrate;
an intermediate oxide layer containing MgO·SiO₂ coated on the first metal oxide layer; and
a second metal oxide layer coated on the intermediate oxide layer,
wherein the glass flake substrate
has a D₁₀ value in a range from 40 to 80 µm, a D₅₀ value in a range from 160 to 250 µm, and a D₉₀ value in a range from 350 to 600 µm, and has a thickness equal to or greater than 500 nm.

In addition, a method for preparing a pearlescent pigment according to another embodiment of the present disclosure for achieving the above purpose includes: (a) mixing a substrate containing glass flakes having a D₁₀ value in a range from 40 to 80 µm, a D₅₀ value in a range from 160 to 250 µm, and a D₉₀ value in a range from 350 to 600 µm and having a thickness equal to or greater than 500 nm to purified water (DIwater) and then stirring and dispersing the substrate to form a suspension; (b) titrating a first soluble inorganic metal salt solution to the suspension in the step (a) and then hydrolyzing the first soluble inorganic metal salt solution to coat a surface of the flakes with a first metal oxide layer; (c) titrating a soluble inorganic salt solution containing MgO·SiO₂ to the suspension in the step (b) and then hydrolyzing the soluble inorganic salt solution to coat a surface of the first metal oxide layer with an intermediate oxide layer; and (d) titrating a second soluble inorganic metal salt solution to the suspension in the step (c) and then hydrolyzing the second soluble inorganic metal salt solution to coat a surface of the intermediate oxide layer with a second metal oxide layer.

### TECHNICAL EFFECTS

The pearlescent pigment according to the present disclosure may use the glass flake with the limited size distribution as the substrate, and may be formed as the multiple metal oxide layers including the low-refractive index material layer between the high-refractive index material layers are coated on the substrate, thereby achieving the improved sparkling effect in addition to the properties such as the high-luminance, the high-gloss, and the high-chroma.

In addition, as the pearlescent pigment according to the present disclosure uses the glass flake having the limited size distribution as the substrate, the wide range of color change based on the change in the observation viewing angle may be realized.

### BRIEF DESCRIPTION OF DRAWINGS

FIG. 1 is a SEM photograph showing a cross-section of a pearlescent pigment according to an embodiment of the present disclosure.

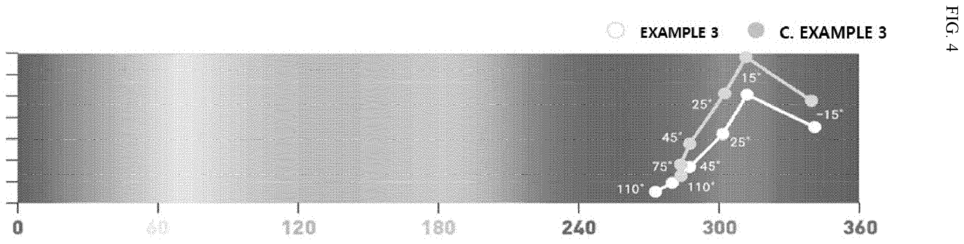

FIGS. 2 to 4 are graphs showing color difference value ranges of pearlescent pigments according to Present Examples of the present disclosure and Comparative Examples.

### DETAILED DESCRIPTIONS

Advantages and features of the present disclosure, and how to achieve them will become apparent with reference to the embodiments described below in detail in conjunction with the accompanying drawings.

However, the present disclosure is not limited to the embodiments as disclosed below, but will be implemented in a variety of different forms. Only these embodiments make the present disclosure complete, and are constructed to fully inform those having common knowledge in the technical field to which the present disclosure belongs of a scope of the disclosure. The scope of the present disclosure is only defined by the scope of the claims.

Hereinafter, a pearlescent pigment having an improved sparkling effect and a method for preparing the same according to an embodiment of the present disclosure will be described in detail.

### Pearlescent pigment

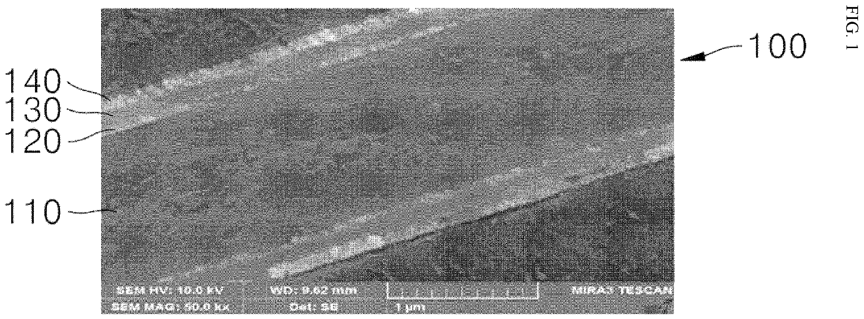

FIG. 1 is a SEM photograph showing a cross-section of a pearlescent pigment according to an embodiment of the present disclosure.

Referring to FIG. 1, a pearlescent pigment 100 according to an embodiment of the present disclosure includes a glass flake substrate 110, a first metal oxide layer 120 on the substrate 110, an intermediate oxide layer 130 made of MgO·SiO₂ on the first metal oxide layer 120, and a second metal oxide layer 140 on the intermediate oxide layer 130.

The pearlescent pigment according to the present disclosure uses the glass flake substrate. In particular, the glass flake substrate has a D₁₀ value in a range from 40 to 80 µm, a D₅₀ value in a range from 160 to 250 µm, and a D₉₀ value in a range from 350 to 600 µm, and has a thickness equal to or greater than 500 nm.

Here, D₁₀, D₅₀, and D₉₀ refer to an average particle diameter in a 10 % area, an average particle diameter in a 50 % area (i.e., a whole average particle diameter), and an average particle diameter in a 90 % area, respectively.

Existing pearlescent pigments used several platelet-shaped substrates such as synthetic mica along with glass flakes. However, the existing platelet-shaped substrates were able to achieve a certain color intensity, but because of lack of user research and understanding on properties, a size, and a thickness of the substrate, existing pigments using the existing platelet-shaped substrates did not exhibit a sparkling effect and had a problem in that a range of a color change based on a change in an observation viewing angle is narrow.

In the present disclosure, it was confirmed that the pearlescent pigment may have an improved sparkling effect by using the glass flake substrate having the D₁₀ value in the range from 40 to 80 µm, the D₅₀ value in the range from 160 to 250 µm, and the D₉₀ value in the range from 350 to 600 µm and having the thickness equal to or greater than 500 nm.

The glass flake substrate according to the present disclosure has the D₁₀ value in the range from 40 to 80 µm, the D₅₀ value in the range from 160 to 250 µm, and the D₉₀ value in the range from 350 to 600 µm. Accordingly, the pigment according to the present disclosure may have the high color intensity and the sparkling effect, and may render various colors based depending on the viewing angle.

In addition, the glass flake substrate according to the present disclosure has the thickness equal to or greater than 500 nm with the above-described size distribution. Accordingly, the pigment according to the present disclosure may have the high color intensity and the sparkling effect, and may render the various colors based depending on the viewing angle. Preferably, the glass flake substrate according to the present disclosure may have a thickness of 1 to 6 µm.

All flake substrates of a glass component may be used for the glass flake substrate according to the present disclosure. Preferably, the glass flake substrate may contain borosilicate or borosilicate doped with at least one of Ti, Zn, and Ca.

The pearlescent pigment 100 is formed by coating the first metal oxide layer 120/the intermediate oxide layer 130/the second metal oxide layer 140 on the substrate 110.

In this regard, the first and second metal oxide layers 120 and 140 may refer to metal oxide layers having high refractive indices that are higher than that of the intermediate oxide layer 130 made of MgO·SiO₂, and may be, preferably, formed as oxide layers with TiO₂ and Fe₂O₃ as main components.

The intermediate oxide layer 130 may be made of a metal oxide having a refractive index 'n' equal to or smaller than 1.8, and may be formed using a metal oxide including MgO·SiO₂ in the present disclosure.

As a result, in the pearlescent pigment 100, metal oxide layers having a high refractive index/a low refractive index/a high refractive index are formed on a surface of the platelet-shaped substrate 110. Preferably, (TiO2 or Fe2O3)/(MgO·SiO2)/(TiO2 or Fe2O3) may be coated on the substrate.

In one example, each of the first and second metal oxide layers 120 and 140 and the intermediate oxide layer 130 is preferably coated with a thickness in a range from 20 nm to 500 nm.

More specifically, thicknesses of the first and second metal oxide layers 120 and 140 are preferably in a range from 30 nm to 130 nm, and a thickness of the intermediate oxide layer 130 is preferably in a range from 120 to 300 nm.

The pearlescent pigment 100 differs in color observed with the naked eye based on a sum of the respective thicknesses of the first and second metal oxide layers 120 and 140 and the intermediate oxide layer 130 or a ratio of the respective thicknesses. However, it is difficult to render the color when the thicknesses of the layers are out of the above range.

The pearlescent pigment 100 according to the embodiment of the present disclosure may be advantageously used for applications in which the pearlescent pigment is used, for example, for various purposes such as coloring in many industrial fields such as various paints, inks for printing, flooring, wallpaper, special paper, plastics, leather, accessories, cosmetics, ceramics, artificial marble, and the like, and may have the improved sparkling effect with a high-chroma color.

### Method for preparing pearlescent pigment

A method for preparing a pearlescent pigment according to the present disclosure includes:
(a) mixing a substrate containing glass flakes having a D₁₀ value in a range from 40 to 80 µm, a D₅₀ value in a range from 160 to 250 µm, and a D₉₀ value in a range from 350 to 600 µm and having a thickness equal to or greater than 500 nm to purified water (DIwater) and then stirring and dispersing the substrate to form a suspension;
(b) titrating a first soluble inorganic metal salt solution to the suspension in step (a) and then hydrolyzing the first soluble inorganic metal salt solution to coat a surface of the flakes with a first metal oxide layer;
(c) titrating a soluble inorganic salt solution containing MgO·SiO₂ to the suspension in step (b) and then hydrolyzing the soluble inorganic salt solution to coat a surface of the first metal oxide layer with an intermediate oxide layer; and
(d) titrating a second soluble inorganic metal salt solution to the suspension in step (c) and then hydrolyzing the second soluble inorganic metal salt solution to coat a surface of the intermediate oxide layer with a second metal oxide layer.

### Suspension formation

In the suspension forming step, the suspension is formed by mixing the glass flake substrate of a certain size used as the substrate with the purified water (DIwater) and then stirring and dispersing the substrate.

Properties of the glass flake substrate are as described above.

In addition, it is preferable to mix the substrate with the purified water such that a content of a substrate solid is in a range from 5 to 20 % by weight in the suspension.

When the content of the solid is smaller than 5 % by weight, the subsequent oxide layer formation reaction may not occur or may be insufficiently performed. In addition, when the content of the solid exceeds 20 % by weight, a reaction efficiency may decrease.

When the suspension for preparing the pigment is formed as above, a temperature of the suspension is first raised to a temperature in a range from 60 to 90 °C. The reason why the suspension is heated as such is that when the temperature of the suspension is lower than 60 °C, a coating state is not uniform, and a size and a shape of a coating material are very irregular. In addition, when the temperature of the suspension exceeds 90 °C, the reaction for the coating may occur violently, and thus, a rough coating layer may be formed.

In this regard, as described above, because the pigment cannot have high chroma when the state of the coating layer is non-uniform, it is preferable to maintain the above temperature range. In addition, the temperature range as described above may be equally applied to all of reactions for forming the first and second metal oxide layers and the intermediate oxide layer below.

### First metal oxide layer formation

After completing the suspension preparation and temperature raising steps as described above, next, the first soluble inorganic metal salt solution is titrated to the suspension and then is hydrolyzed to coat the surface of the flakes with the first metal oxide layer.

In this regard, an inorganic metal salt is made of one selected from a group consisting of SnCl₄, TiCl₄, TiOCl₂, TiOSO₄, FeCl₃, FeSO₄, SiCl₄, ZrOCl₂, Na₂O·SiO₂·5H₂O, MnCl₂, MgCl₂, AlCl₃, and CoCl₂, or a mixture of one or more of those.

Then, the first soluble inorganic metal salt solution in which the inorganic metal salt is dissolved is added dropwise to the suspension so as to be hydrolyzed.

In this regard, a pH value of the suspension is in a range from 1 to 9. When the pH is smaller than 1, the coating of the first metal oxide layer is not normally performed, and when the pH exceeds 9, the coating material has non-uniform and very irregular size and shape. Therefore, because the coating state becomes very rough, the pigment cannot have the high chroma.

In addition, after the injection of the solution is completed while the pH value is maintained constant, a process of refluxing the suspension for 10 to 30 minutes is performed.

In this regard, the pH value is maintained constant at a level at which a coverage of the first metal oxide layer formed on the surface of the substrate is in a range from 1 to 50 %, and the reflux process reduces an impact caused by the reaction pH and allows the coating material to be sufficiently coated on the surface.

Therefore, when a reflux time is shorter than 10 minutes, the sufficient coverage cannot be obtained and the impact may be applied to the substrate, causing cracks. In addition, when the reflux time exceeds 30 minutes, breaking of the substrate itself or separation of the coating layer caused by the stirring may occur.

### Intermediate oxide layer: MgO·SiO₂ layer formation

Via the above process, the temperature of the suspension in which the solid coated with the first metal oxide layer is mixed onto the surface of the substrate is raised again to a temperature in the range from 60 to 90 °C. In this regard, the temperature range is a temperature range for forming an optimal coating layer as described above.

Next, after titrating the soluble inorganic salt solution containing MgO·SiO₂ to the heated suspension, the soluble inorganic salt solution is hydrolyzed to coat the surface of the first metal oxide layer with MgO·SiO₂. In the present disclosure, a layer formed by coating MgO·SiO₂ alone or coating other oxides with MgO·SiO₂ is referred to as an oxide layer.

In this regard, the soluble inorganic salt solution is made of one selected from a group consisting of water glass, MgCl₂, silicate, AlCl₃, KCl₃, and boric acid, or a mixture of one or more of those.

Then, the pH value of the suspension is in a range from 4 to 14. When the pH is smaller than 4, the coating of the oxide layer is not normally performed, and the coating material has non-uniform and very irregular size and shape, so that the pigment cannot have the high chroma.

In addition, after the injection of the solution is completed while the pH value is maintained constant, a process of refluxing the suspension for 30 to 60 minutes is performed.

In this regard, the pH value is preferably adjusted such that the coverage of the oxide layer formed on the surface of the substrate is in a range from 1 to 30 % in a case of high-chroma and high-gloss pigment, and the coverage of the oxide layer is in a range from 30 to 90 % in a case of multi-color pigment.

In the case of multi-color use, the coverage of the oxide layer may be increased up to 3 times the coverage of the oxide layer in the case of glossy pigment. Accordingly, a total weight ratio of the oxide layer may vary for the pigment having the high-gloss and high-chroma properties and for the pigment having the multi-colors.

The pigment having the high-gloss and high-chroma properties has an optimal performance when the total weight ratio of the oxide layer is in a range from 5 to 10 % by weight based on a total weight of the completed pigment. That is, when the ratio of the oxide layer was smaller than 5 % by weight of the total weight of the pigment, the high-gloss property was deteriorated, and when the ratio of the oxide layer was greater than 10 % by weight, the high-chroma property was deteriorated.

In addition, the pigment having the multi-colors has an optimal performance when the total weight ratio of the oxide layer is in a range from 5 to 35 % by weight based on the total weight of the completed pigment. That is, when the ratio of the oxide layer was smaller than 5 % by weight of the total weight of the pigment, there was a problem that the pigment exhibits a single color, and when the ratio of the oxide layer exceeds 35 % by weight, the color change properties were deteriorated.

Therefore, a preferred content ratio of the oxide layer according to the present disclosure may be in the range from 5 to 35 % by weight, but may not always be limited thereto, and may vary depending on the type of substrate, the material to be coated, the coating thickness, and the like.

The oxide layer according to the present disclosure formed as described above may have MgO·SiO₂ as a main component, and may further contain one selected from a group consisting of SiO₂, MgO·Al₂O₃, K₂O·SiO₂, and Mg₂SiO₄, or a mixture of one or more of those.

Such an oxide layer may serve as a low-refractive index layer in the pigment, and may solve existing problems such as cracks that occur when only SiO₂ is formed as a conventional low refractive index layer. In addition, when the MgO·SiO₂ oxide layer is used, it may be easy to adjust a thickness of the low-refractive index layer and may be easy to exhibit the high-gloss, high-chroma, and multi-color properties.

### Second metal oxide layer formation

In this regard, in the present disclosure, to protect the intermediate oxide layer and improve the properties such as the high-gloss property, the second metal oxide layer is coated on top of the oxide layer, and such process is performed in the same manner as the first metal oxide coating process.

Next, the suspension that has been coated with the final second metal oxide layer is filtered, washed with deionized water and dried, and screened by calcining a residue to complete the preparation of the pearlescent pigment according to the present disclosure.

As described above, the pearlescent pigment according to the present disclosure coated with the multiple layers of a 7-layer structure includes the low-refractive index layer and the high-refractive index layer formed on top of the transparent substrate layer, and has the high-gloss, high-chroma, and excellent multi-color properties.

In addition, in the present disclosure, the pearlescent pigment may have the improved sparkling effect by using the glass flake substrate having the D₁₀ value in the range from 40 to 80 µm, the D₅₀ value in the range from 160 to 250 µm, and the D₉₀ value in the range from 350 to 600 µm and having the thickness equal to or greater than 500 nm.

Hereinafter, the sparkling effect along with the high-gloss, high-chroma, and multi-color properties based on the use of the glass flake substrate and the multi-layer structure will be described.

### Present Example

Hereinafter, a composition and an operation of the present disclosure will be described in more detail with preferred Present Examples of the present disclosure. However, these are presented as preferred examples of the present disclosure and cannot be construed as limiting the present disclosure in any way.

Contents not described herein may be technically inferred by those skilled in the art, so that descriptions thereof will be omitted.

### <Present Example 1>

100 g of borosilicate flakes having a size distribution of D₁₀ 65.334 µm, D₅₀ 183.040 µm, and D₉₀ 412.243 µm and a thickness of 1.2 µm were added to 1.5 L demineralized water and then stirred to form a slurry. Next, the slurry was heated to 85 °C, and then a HCl solution was added thereto when the temperature of 85 °C was reached, to adjust a pH of the slurry to 2.5.

The size distribution of the borosilicate flakes was measured using a particle size analyzer (Master Sizer 2000 from Malvern Instruments). In addition, the average thickness of the borosilicate flakes was measured via observation using an electron microscope.

Next, 100 g of a SnCl₄ solution (SnCl₄ content: 10.0 % by weight) was weighed and titrated to the slurry at a constant rate over 1 hour while maintaining the pH constant with a 10 to 50 % NaOH diluted solution. After the titration, the slurry was refluxed for 10 minutes.

Next, 120 g of a TiCl₄ solution (TiCl₄ content: 30.0 % by weight) was weighed and titrated to the slurry at a constant rate over 4 hours while maintaining the pH constant with the 10 to 50 % NaOH diluted solution. After the titration, the slurry was refluxed for 10 minutes, and then the pH thereof was adjusted to 6.0 with a 10 to 30 % NaOH diluted solution.

Next, 2200 g of a MgO·SiO₂ solution (MgO·SiO₂ content 15.0 % by weight) was weighed and titrated to the slurry at a constant rate over 10 hours while maintaining the pH of 6.0 constant with an HCl solution. The pH of the slurry was adjusted to 2.5 by adding the HCl solution thereto and then the slurry was stirred for additional 15 minutes and refluxed.

Next, 200 g of a SnCl₄ solution (SnCl₄ content: 10.0 % by weight) was weighed and titrated to the slurry at a constant rate over 2 hours while maintaining the pH constant with the 10 to 50 % NaOH diluted solution. After the titration, the slurry was refluxed for 10 minutes.

Next, 120 g of the TiCl₄ solution (TiCl₄ content: 30.0 % by weight) was weighed and titrated to the slurry at a constant rate over 4 hours while maintaining the pH constant with the 10 to 50 % NaOH diluted solution. After the titration, the slurry was refluxed for 10 minutes.

After the refluxing, the final slurry was filtered and dehydrated, washed twice with the demineralized water, and dried at 120 °C for 10 hours to obtain an intermediate product as a powdery residue.

Finally, 11 g of the obtained intermediate product was calcined at 800 °C for 12 minutes to obtain a gold-green tone powder.

### <Present Example 2>

100 g of borosilicate flakes having a size distribution of D₁₀ 71.758 µm, D₅₀ 193.732 µm, and D₉₀ 429.438 µm and a thickness of 1.3 µm were added to 1.5 L demineralized water and then stirred to form a slurry. Next, the slurry was heated to 85 °C, and then a HCl solution was added thereto when the temperature of 85 °C was reached, to adjust a pH of the slurry to 2.5.

The size distribution of the borosilicate flakes was measured using a particle size analyzer (Master Sizer 2000 from Malvern Instruments). In addition, the average thickness of the borosilicate flakes was measured via observation using an electron microscope.

Next, 100 g of a SnCl₄ solution (SnCl₄ content: 10.0 % by weight) was weighed and titrated to the slurry at a constant rate over 1 hour while maintaining the pH constant with a 10 to 50 % NaOH diluted solution. After the titration, the slurry was refluxed for 10 minutes.

Next, 120 g of a TiCl₄ solution (TiCl₄ content: 30.0 % by weight) was weighed and titrated to the slurry at a constant rate over 4 hours while maintaining the pH constant with the 10 to 50 % NaOH diluted solution. After the titration, the slurry was refluxed for 10 minutes, and then the pH thereof was adjusted to 6.0 with a 10 to 30 % NaOH diluted solution.

Next, 1100 g of a MgO·SiO₂ solution (MgO·SiO₂ content 15.0 % by weight) was weighed and titrated to the slurry at a constant rate over 8 hours while maintaining the pH of 6.0 constant with an HCl solution. The pH of the slurry was adjusted to 2.5 by adding the HCl solution thereto and then the slurry was stirred for additional 15 minutes and refluxed.

Next, 200 g of a SnCl₄ solution (SnCl₄ content: 10.0 % by weight) was weighed and titrated to the slurry at a constant rate over 2 hours while maintaining the pH constant with the 10 to 50 % NaOH diluted solution. After the titration, the slurry was refluxed for 10 minutes.

Next, 120 g of the TiCl₄ solution (TiCl₄ content: 30.0 % by weight) was weighed and titrated to the slurry at a constant rate over 4 hours while maintaining the pH constant with the 10 to 50 % NaOH diluted solution. After the titration, the slurry was refluxed for 10 minutes.

After the refluxing, the final slurry was filtered and dehydrated, washed twice with the demineralized water, and dried at 120 °C for 10 hours to obtain an intermediate product as a powdery residue.

Finally, 11 g of the obtained intermediate product was calcined at 800 °C for 12 minutes to obtain a red-gold tone powder.

### <Present Example 3>

100 g of borosilicate flakes having a size distribution of D₁₀ 75.708 µm, D₅₀ 177.288 µm, and D₉₀ 384.897 µm and a thickness of 1.1 µm were added to 1.5 L demineralized water and then stirred to form a slurry. Next, the slurry was heated to 85 °C, and then a HCl solution was added thereto when the temperature of 85 °C was reached, to adjust a pH of the slurry to 2.5.

The size distribution of the borosilicate flakes was measured using a particle size analyzer (Master Sizer 2000 from Malvern Instruments). In addition, the average thickness of the borosilicate flakes was measured via observation using an electron microscope.

Next, 100 g of a SnCl₄ solution (SnCl₄ content: 10.0 % by weight) was weighed and titrated to the slurry at a constant rate over 1 hour while maintaining the pH constant with a 10 to 50 % NaOH diluted solution. After the titration, the slurry was refluxed for 10 minutes.

Next, 150 g of a TiCl₄ solution (TiCl₄ content: 30.0 % by weight) was weighed and titrated to the slurry at a constant rate over 4 hours while maintaining the pH constant with the 10 to 50 % NaOH diluted solution. After the titration, the slurry was refluxed for 10 minutes, and then the pH thereof was adjusted to 6.0 with a 10 to 30 % NaOH diluted solution.

Next, 3000 g of a MgO·SiO₂ solution (MgO·SiO₂ content 15.0 % by weight) was weighed and titrated to the slurry at a constant rate over 15 hours while maintaining the pH of 6.0 constant with an HCl solution. The pH of the slurry was adjusted to 2.5 by adding the HCl solution thereto and then the slurry was stirred for additional 15 minutes and refluxed.

Next, 200 g of a SnCl₄ solution (SnCl₄ content: 10.0 % by weight) was weighed and titrated to the slurry at a constant rate over 2 hours while maintaining the pH constant with the 10 to 50 % NaOH diluted solution. After the titration, the slurry was refluxed for 10 minutes.

Next, 150 g of the TiCl₄ solution (TiCl₄ content: 30.0 % by weight) was weighed and titrated to the slurry at a constant rate over 4 hours while maintaining the pH constant with the 10 to 50 % NaOH diluted solution. After the titration, the slurry was refluxed for 10 minutes.

After the refluxing, the final slurry was filtered and dehydrated, washed twice with the demineralized water, and dried at 120 °C for 10 hours to obtain an intermediate product as a powdery residue.

Finally, 11 g of the obtained intermediate product was calcined at 800 °C for 12 minutes to obtain a violet-orange tone powder.

### Comparative Examples 1 to 3

### <Comparative Example 1>

Except for using 100 g of synthetic mica flakes having a size distribution of D₁₀ 7.259 µm, D₅₀ 18.366 µm, and D₉₀ 36.876 µm and having a thickness of 0.35 µm, a pigment powder according to Comparative Example 1 was obtained in the same manner as that in Present Example 1.

### <Comparative Example 2>

Except for using 100 g of synthetic mica flakes having a size distribution of D₁₀ 7.4759 µm, D₅₀ 19.307 µm, and D₉₀ 37.991 µm and having a thickness of 0.37 µm, a pigment powder according to Comparative Example 2 was obtained in the same manner as that in Present Example 2.

### <Comparative Example 3>

Except for using 100 g of synthetic mica flakes having a size distribution of D₁₀ 10.672 µm, D₅₀ 21.476 µm, and D₉₀ 39.665 µm and having a thickness of 0.39 µm, a pigment powder according to Comparative Example 3 was obtained in the same manner as that in Present Example 3.

### Physical property evaluation of Present Examples and Comparative Examples

### 1. Glossiness evaluation

Glossiness of Present Examples and Comparative Examples were evaluated as follows, and the results are written in Tables 1 and 2 below.

The glossiness was measured in two schemes as follows.
1) Clear coats respectively containing the pigments according to Present Examples and Comparative Examples were prepared and sprayed, and then gloss values of the pigments according to Present Examples and Comparative Examples were evaluated. The evaluation results are shown in Table 1 below.
2) NA clears containing the pigments according to Present Examples and Comparative Examples were prepared and applied to cover paper in a draw-down scheme, and then gloss values of the pigments according to Present Examples and Comparative Examples was evaluated. The evaluation results are shown in Table 2 below.

**[Table 1]**

| | 20 ° | 60 ° | 85 ° |
|---|---|---|---|
| Present Example 1 | 120.67 | 113.96 | 100.42 |
| Present Example | 117.97 | 118.93 | 97.24 |
| 2 | | | |
| Present Example 3 | 114.52 | 112.52 | 105.72 |
| Comparative Example 1 | 109.77 | 108.77 | 97.60 |
| Comparative Example 2 | 111.68 | 109.05 | 94.10 |
| Comparative Example 3 | 112.88 | 109.60 | 101.18 |

**[Table 2]**

| | 20 ° | 60 ° | 85 ° |
|---|---|---|---|
| Present Example 1 | 30.3 | 66.22 | 52.68 |
| Present Example 2 | 25.78 | 74.96 | 53.78 |
| Present Example 3 | 16.58 | 59.18 | 50.54 |
| Comparative Example 1 | 21.56 | 54.62 | 42.66 |
| Comparative Example 2 | 15.72 | 55.44 | 39.08 |
| Comparative Example 3 | 13.52 | 55.58 | 46.56 |

As may be seen from the above results, the pigments according to Present Examples of the present disclosure have greater gloss values than that according to Comparative Examples. It may be identified that such results are resulted from differences in the substrate.

### 2. Sparkling effect evaluation

To identify sparkling effects of Present Examples and the Comparative Examples, Dsparkle values were measured, and the results are written in Table 3 below.

The Dsparkle values were measured using a BYK-mac i 23mm.

A Dsparkle value of the pigment according to Present Example 1 was measured based on a Dsparkle value of 1 of Comparative Example 1, and a Dsparkle value of the pigment according to Present Example 2 was measured based on a Dsparkle value of 1 of Comparative Example 2, and a Dsparkle value of the pigment according to Present Example 3 was measured based on a Dsparkle value of 1 of Comparative Example 3.

**[Table 3]**

| | 15 | 45 | 75 |
|---|---|---|---|
| Present Example 1 | 27.61 | 19.54 | 4.48 |
| Present Example 2 | 26.9 | 12.68 | 6.82 |
| Present Example 3 | 20.14 | 19.65 | 7.22 |

As may be seen from the above results, the Dsparkle values of the pigments according to Present Examples of the present disclosure are significantly greater than those of the pigments according to Comparative Examples. It may be identified that such results are resulted from differences in the substrate, and it may be seen that Present Examples have excellent sparkling effects compared to Comparative Examples.

### 3. Color difference value range evaluation

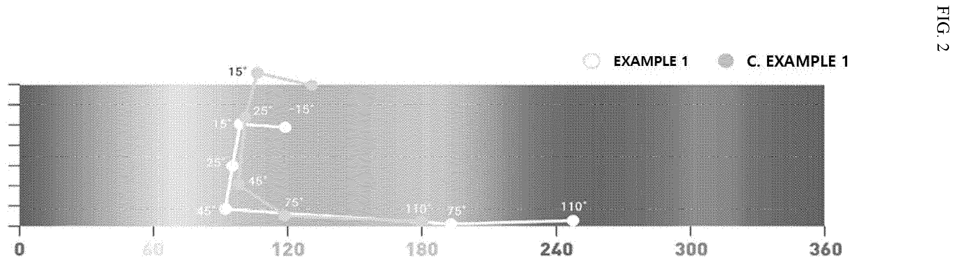

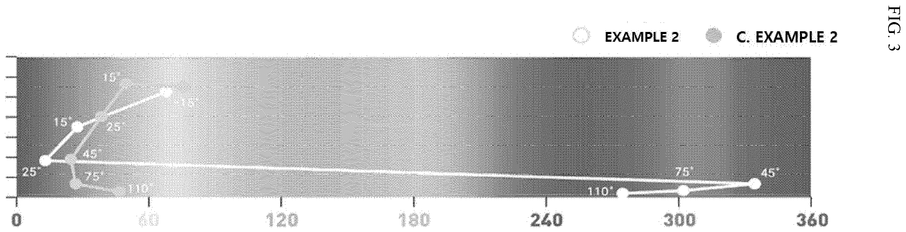

Color difference value ranges according to Present Examples and Comparative Examples were evaluated, and the results are written in Table 4 below. In addition, the results of the color difference value ranges according to Present Examples and Comparative Examples are shown in graphs (see FIGS. 1 to 3).

Color difference values were measured using a BYK-mac i 23mm.

**[Table 4]**

| | h | | | | | |
|---|---|---|---|---|---|---|
| | -15 | 15 | 25 | 45 | 75 | 110 |
| Present Example 1 | 118.94 | 98.71 | 95.27 | 92.1 | 192.85 | 247.53 |
| Comparative Example 1 | 130.76 | 106.35 | 101.11 | 97.87 | 118.52 | 180 |
| Present Example 2 | 67.64 | 27.5 | 12.99 | 334.32 | 302.03 | 274.32 |
| Comparative Example 2 | 75.27 | 49.55 | 38.33 | 24.6 | 26.7 | 46.65 |
| Present Example 3 | 340.93 | 312.01 | 301.64 | 287.37 | 280 | 272.86 |
| Comparative Examples | 339.57 | 311.54 | 302.42 | 287.53 | 283.57 | 283.73 |

| | | | | | | |
|---|---|---|---|---|---|---|
| With the results of the color difference value ranges, how various colors the corresponding pigment may render depending on the viewing angle may be identified. | | | | | | |

Referring to Table 4 and FIGS. 1 to 3, it may be seen that the color difference value range of the pigment according to Comparative Example falls within the color difference value range of the pigment according to Present Example. In other words, it may be seen that the pigment according to Present Example renders a wider range of colors than the pigment according to Comparative Example, and the pigment according to Present Example renders various colors depending on the viewing angle compared to the pigment according to Comparative Example.

As may be seen from the above results, the pigment according to Present Example of the present disclosure has the wider color difference value range than the pigment according to Comparative Example. It may be identified that such results are resulted from differences in the substrate, and the fact that the color difference value range is wide may be seen that Present Example may render the various colors depending on the viewing angle.

Although embodiments of the present disclosure have been described with reference to the accompanying drawings, the present disclosure may not be limited to the above embodiments and may be modified in various different forms. Those skilled in the art to which the present disclosure belongs will be able to understand that the present disclosure may be implemented in other specific forms without changing the technical spirit or essential characteristics thereof. Therefore, the embodiments described above should be understood as illustrative in all respects and not limiting.

The present disclosure is an invention derived from research on development and commercialization of a technology for multi-layer coating of metal oxides with a flip flop effect in the 2020 Chungbuk materials • parts • equipment technology development support project of Chungcheongbuk-do and Chungbuk Innovation Institute of Science & Technology.

## Claims

1. A pearlescent pigment comprising:
a glass flake substrate;
a first metal oxide layer coated on the substrate;
an intermediate oxide layer containing MgO·SiO₂ coated on the first metal oxide layer; and
a second metal oxide layer coated on the intermediate oxide layer,
wherein the glass flake substrate has a D₁₀ value in a range from 40 to 80 µm, a D₅₀ value in a range from 160 to 250 µm, and a D₉₀ value in a range from 350 to 600 µm, and has a thickness equal to or greater than 500 nm.

2. The pearlescent pigment of claim 1, wherein the glass flake substrate contains borosilicate or borosilicate doped with at least one selected from a group consisting of Ti, Zn, and Ca.

3. The pearlescent pigment of claim 1, wherein the glass flake substrate has a thickness in a range from 1 to 6 µm.

4. The pearlescent pigment of claim 1, wherein the intermediate oxide layer further contains one or a mixture of at least two selected from a group consisting of SiO₂, MgO·Al₂O₃, K₂O·SiO₂, and Mg₂SiO₄.

5. The pearlescent pigment of claim 1, wherein the pigment is used in at least one of a paint, a printing ink, flooring, wallpaper, special paper, plastic, leather, accessories, cosmetics, ceramic, and artificial marble.

6. A method for preparing a pearlescent pigment, the method comprising:
(a) mixing a substrate containing glass flakes having a D₁₀ value in a range from 40 to 80 µm, a D₅₀ value in a range from 160 to 250 µm, and a D₉₀ value in a range from 350 to 600 µm and having a thickness equal to or greater than 500 nm to purified water (DIwater) and then stirring and dispersing the substrate to form a suspension;
(b) titrating a first soluble inorganic metal salt solution to the suspension in the step (a) and then hydrolyzing the first soluble inorganic metal salt solution to coat a surface of the flakes with a first metal oxide layer;
(c) titrating a soluble inorganic salt solution containing MgO·SiO₂ to the suspension in the step (b) and then hydrolyzing the soluble inorganic salt solution to coat a surface of the first metal oxide layer with an intermediate oxide layer; and
(d) titrating a second soluble inorganic metal salt solution to the suspension in the step (c) and then hydrolyzing the second soluble inorganic metal salt solution to coat a surface of the intermediate oxide layer with a second metal oxide layer.

7. The method of claim 6, wherein the suspension in the step (a) has a solid content in a range from 5 to 20 % by weight.

8. The method of claim 6, wherein the suspensions in the steps (b) to (d) are maintained at a temperature in a range from 60 to 90 °C.

9. The method of claim 6, wherein each of the first soluble inorganic metal salt solution and the second soluble inorganic metal salt solution further contains one or a mixture of at least two selected from a group consisting of SnCl₄, TiCl₄, TiOCl₂, TiOSO₄, FeCl₃, FeSO₄, SiCl₄, ZrOCl₂, Na₂O·SiO₂·5H₂O, MnCl₂, MgCl₂, AlCl₃, and CoCl₂.

10. The method of claim 6, wherein the soluble inorganic salt solution further contains one or a mixture of at least two selected from a group consisting of water glass, MgCl₂, silicate, AlCl₃, KCl₃, and boric acid.

11. The method of claim 6, wherein the intermediate oxide layer further contains one or a mixture of at least two selected from a group consisting of SiO₂, MgO·Al₂O₃, K₂O·SiO₂, and Mg₂SiO₄.

12. The method of claim 6, wherein the intermediate oxide layer is contained in an amount of 5 to 35 % by weight based on 100 % by weight of a total composition of the pigment.

13. The method of claim 6, wherein a pH value of the suspension in the step (b) or the step (d) is adjusted to be in a range from 1 to 9, and the suspension is refluxed for 10 to 30 minutes after the titration of the solution is completed.

14. The method of claim 6, wherein a pH value of the suspension in the step (c) is adjusted to be in a range from 4 to 14, and the suspension is refluxed for 30 to 60 minutes after the titration of the solution is completed.
